# EUROPEAN PATENT APPLICATION

(11) **EP 4 589 262 A1**
(43) Date of publication of application: **23.07.2025**
(21) Application number: 23865478.4
(22) Date of filing: 11.09.2023
(51) Int. Cl.: G01D 9/00

(54) **MEASURING DEVICE, MEASURING SYSTEM, AND MEASURING DEVICE CONTROL METHOD**

(30) Priority: 13.09.2022 JP 2022145435
(71) Applicant: T&D Corporation, Matsumoto-shi, Nagano 390-0852 (JP)
(72) Inventor: MOROZUMI Yuta, Tokyo 150-0044 (JP); SEGI Chiaki, Matsumoto-shi Nagano 390-0852 (JP)
(74) Representative: Liesegang, Eva
(86) International application number: PCT/JP2023/033058
(87) International publication number: WO 2024/058125

(57) **Abstract**

A measuring device 1 includes: a measured data accumulating unit 41 for writing measured data MD to a record 30; an identification code assignment range selection unit 43 which, when an identification code IC is newly obtained, if the identification code IC has not been written in an identification code field 35 of the record immediately prior to a most recent measurement cycle, prompts an operator to select whether to set an identification code assignment range to (A) single measured data or (B) a plurality of measured data; an identification code individual assignment unit 45 for writing the identification code IC to the record of the most recent measurement cycle in case of (A), and an identification bulk assignment unit 47 for writing the identification code to consecutive records to which the identification code IC has not yet been written in case of (B). According to the present invention, various modes of operation can be implemented without impairing the ease of the operation, and identification codes can be assigned to the single or plurality of measured data in a versatile manner.

## Description

### Technical Field

The present invention relates to a measuring device, a measuring system and a measuring device control method, and more particularly to a technique for associating obtained measurement data with an identification code.

### Background Art

As a device for measuring a temperature of a measured object, an electronic thermometer, an electronic body thermometer or the like (hereinafter such a thermometer also being simply referred to as "thermometer") has been popularly used. The thermometer has a relatively simple specification. When a user directs the portion of the thermometer near the temperature sensor toward a measured object and presses the measurement button, the temperature measurement begins, and when the measurement is properly completed, a beep sound is emitted and the measured data is displayed on a display equipment.

There has also been known a measuring device that records data obtained by measurement and an identification code (for example, see patent literature 1 and patent literature 2). The measuring devices described in patent literature 1 and patent literature 2 each measure a temperature of a measured object, obtain identification information from a barcode, a magnetic card, various kinds of electronic tags, a key input and the like, and record an identification code, an identification number or the like (referred to as "identification code" representatively) that corresponds to the identification information in an associated manner with measured data obtained by the measurement.

Recently, there has been an increasing number of cases in which it is required to manage the assignment of one identification code to a plurality of identification codes obtained from multiple measurements. For example, at the time of measuring a temperature of an object by measuring a temperature of a surface of an object, in a case in which the measured object is a relatively large object such as a shipping container, there is a possibility that portions of the object in which a temperature measurement is performed respectively exhibit different temperatures. In such a situation, there is a case in which temperature management is required to be performed in such a manner that a temperature is measured at a plurality of portions on a surface of an object respectively so as to obtain measured data at the respective portions of the object. One identification code unique to the container is assigned and recorded with respect to a group formed of the plurality of these measured data and, then, the temperature management is performed by calculating an average value of the measured data for each container later. Further, there is also a case in which it is required to perform the temperature control similar to the above by measuring a temperature at a plurality of places in a space such as a conference room or a warehouse.

### Citation List

### Patent Literature

[Patent Literature 1] JP-A-2004-157803
[Patent Literature 2] JP-A-11-113856
[Patent Literature 3] JP-A-2001-353157

### Summary of Invention

### Technical Problem

However, the measuring devices described in patent literature 1 and patent literature 2 are provided for controlling measured data and an identification code associated with the measured data as a single pair. Accordingly, in a case in which it is required to assign one identification code to a group formed of a plurality of measured data as described above, in each measurement, it is required to read the identification information, and to assign an identification code based on the identification information as a set. That is, it is necessary to perform also the reading operation of the identification information together with the measurement operation each time and hence, the operations become cumbersome. Further, in a case in which a place where a measurement is performed and a place where a barcode, an electronic tag or the like that contains identification information is disposed are physically separated from each other, the measurement of temperature and reading of identification information cannot be performed at the same operation timing. The above-mentioned operations in such a case become further cumbersome.

On the other hand, patent literature 3 describes a measuring device in which a fingerprint (identification information) of a subject to be measured is read, and the fingerprint is recorded as numerical values (see paragraph [0023] to paragraph [0032], Fig. 5, Fig. 7 and the like in patent literature 3), thereafter, a basal body temperature of the subject to be measured is measured plural times and a plural number of measured data are recorded. The measuring device described in patent literature 3 can manage the assignment of one identification code to the plurality of measured data obtained from multiple measurements.

However, in the measuring device described in patent literature 3, the measured data is directed to the specific unique subjects to be measured and hence, the measured data control having general-use property and flexibility such as assigning an identification code to a group of arbitrary subjects to be measured as needed cannot be performed.

Further, recently, a demand for performing hygienic management and quality management more meticulously has been increased. That is, the number of cases where a user wants to use one measurement device in various operation modes has been increased. Such operation modes include: an operation mode in which only temperature measurement (and its recording) is simply performed; an operation mode in which an identification code is individually assigned to each measured data obtained by performing a single measurement while performing temperature measurement and its recording; an operation mode in which an identification code is assigned to a plurality of measured data while performing temperature measurement and its recording. Further, there also exists a demand that a user wants to use such various operation modes not in a fixed manner but in a dynamically switchable manner. For example, there may be a case in which a shop will appear where the shop wants to perform a management using the same thermometer in such a manner that only body temperature measurement is usually performed for customers when they visit the shop, and when an employee comes to work, the thermometer reads a security card while performing the body temperature measurement of the employee, and a predetermined identification code is assigned to the measured data.

On the other hand, from a viewpoint of a user who wants to use the thermometer in an operation mode in which the only temperature measurement is performed, it is desirable that the operation specification be a simple specification which does not make the user be conscious of mode switching in the same manner as an existing general thermometer or body thermometer.

With respect to various drawbacks described above, not only a device that measures temperature but also devices that measure other quantities have substantially the same drawbacks.

The present invention has been made in view of such circumstances, and it is an object of the present invention to provide a measuring device that can realize various operation modes without impairing the ease of operations, and can realize assigning of an identification code to a single or plural measured data in a general-use form. Further, it is another object of the present invention to provide a measurement system and a method of controlling a measuring device.

### Solution to Problem

According to one aspect of the present invention, there is provided a measuring device for measuring an "amount to be measured" that is an amount indicating a state or a property of a measured object.

The measuring device includes: a measuring unit configured to measure the amount to be measured; an ID reading unit configured to read identification information from outside the measuring device, and configured to output the read identification information as an identification code; a storage unit configured to store a plurality of records: and a control unit configured to control an operation of the measuring device.

A unit of a data structure that includes at least: a measured data field capable of storing measured data obtained from measurement by the measurement unit, an amount of the measured data corresponding to the measurement performed one time; and an identification code field capable of storing the identification code associated with the measured data is defined as one "record".

The control unit includes a measured data accumulating unit configured to instruct the measuring unit to measure the amount to be measured in response to an instruction of an operator, and, configured to write the measured data obtained by the measurement as a part of the record of the storage unit one or more times.

Further, the control unit includes an identification code assignment range selection unit configured to reference the storage unit when the ID acquisition unit newly reads the identification information and obtains the identification code, and configured to, when the storage unit is in a state in which the identification code has not been written in an identification code field of the record that is one cycle prior to the record that corresponds to the most recent measurement cycle, prompts an operator to select either "(A) to assign an identification code to a single measured data" or "(B) to assign an identification code to a plurality of measured data".

Still, further, the control unit includes an identification code individual assignment unit configured, when the operator selects "(A) to assign an identification code to a single measured data", to write the newly obtained identification code only to the record that corresponds to the most recent measurement cycle.

Still further, the control unit includes an identification code bulk assignment unit configured, when the operator selects "(B) to assign an identification code to a plurality of measured data", to collectively write the newly obtained same identification code to each consecutive record that is in a state in which no identification code is written in the identification code field as viewed retroactively in order from the record of the most recent measurement cycle.

According to the measuring device of the present invention, it is possible to provide the measuring device that can realize the various operation modes without impairing the ease of the operation and, at the same time, can realize assigning an identification code to a single or a plurality of measured data in a general-use form.

According to another aspect of the present invention, there is provided a measuring system that includes: the measuring device described above that further includes a communication I/F; and a terminal device connected to the measuring device through communication via the communication I/F. In such a measuring system, in response to a predetermined command, the measuring device transmits a record in which the measured data is written to the terminal device, and the terminal device is configured to receive the record transmitted from the measuring device.

According to still another aspect of the present invention, there is provided a method of controlling a measuring device for measuring an "amount to be measured" that is an amount indicating a state or a property of a measured object. In such a method, the configuration of the measuring device that forms a premise of the method described above has substantially the same configuration as the measuring device described above.

The method of controlling a measuring device includes: a measured data accumulating step; an identification code assignment range selecting step; an identification code individual assigning step; and an identification code bulk assigning step.

In the measured data accumulating step, the control unit executes a set of a measuring process that instructs the measuring unit to measure the amount to be measured in response to an instruction of an operator, and, a measuring data writing process of writing the measured data obtained from the measurement as a part of the record in a predetermined field of the storage unit, one or more times.

In the identification code assignment range selecting step, when the ID acquisition unit newly reads the identification information and obtains the identification code, the control unit references the storage unit and, when the storage unit is in a state in which the identification code has not been written in an identification code field of the record that is one cycle prior to the record that corresponds to the most recent measurement cycle, the control unit prompts an operator to select either "(A) to assign an identification code to a single measured data" or "(B) to assign an identification code to a plurality of measured data".

In the identification code individual assigning step, when the operator selects "(A) to assign an identification code to a single measured data", the control unit writes the newly obtained identification code only to the record that corresponds to the most recent measurement cycle.

In the identification code bulk assignment unit, when the operator selects "(B) to assign an identification code to a plurality of measured data", the control unit collectively writes the newly obtained same identification code to each consecutive record that is in a state in which no identification code is written in the identification code field as viewed retroactively in order from the record of the most recent measurement cycle.

### Brief Description of Drawings

Fig. 1 is a constitutional view illustrating an example of a measuring device 1 and a measuring system 3 that includes the measuring device 1 according to an embodiment 1.
Fig. 2 is a block diagram illustrating one example of the hardware configuration of an information processing device 10 used in embodiments 1 to 3.
Fig. 3 is a block diagram illustrating one example of the functional configuration of the measuring device 1 according to the embodiment 1.
Fig. 4 is a view illustrating a data structure of a record 30 according to the embodiment 1.
Fig. 5 is a view illustrating an operation of a control unit 40, and is also a view illustrating an example of a data writing state to a "table" that constitutes a plurality of records 30.
Fig. 6 is a view illustrating an operation of the control unit 40, and is also a view illustrating an example of a data writing state to the "table" that constitutes the plurality of records 30.
Fig. 7 is a view illustrating an operation of the control unit 40, and is also a view illustrating an example of a data writing state to the "table" that constitutes the plurality of records 30.
Fig. 8 is a flow chart illustrating an example of a method of controlling the measuring device 1 according to the embodiment 1.
Fig. 9 is a view illustrating a measuring device 1a according to the embodiment 1.
Fig. 10 is a view illustrating a measuring device 2 according to an embodiment 2.
Fig. 11 is an example of a sequence diagram illustrating an operation of a measuring system 3 according to an embodiment 3.

### Description of Embodiments

Hereinafter, the description is made with respect to a measuring device, a control method of the measuring device, and a measuring system according to the present invention by referencing drawings. With respect to the symbols that are used in common across the drawings, since the descriptions of these symbols are already provided and can be used in the description of other drawings, the descriptions of the other drawings are omitted.

### [Embodiment 1]

### 1. Measuring device 1 and measuring system 3 that includes measuring device 1

The measuring device 1 according to the embodiment 1 is a device for measuring a quantity representing a state or a property of a measured object MT (hereinafter also referred to as an "amount to be measured"). The same definition is substantially applicable to measuring devices 2, 1a. Before describing the measuring device 1 in detail, to facilitate the understanding of the present invention, first, the summary of the measuring system 3 that includes the measuring device 1 is described hereinafter.

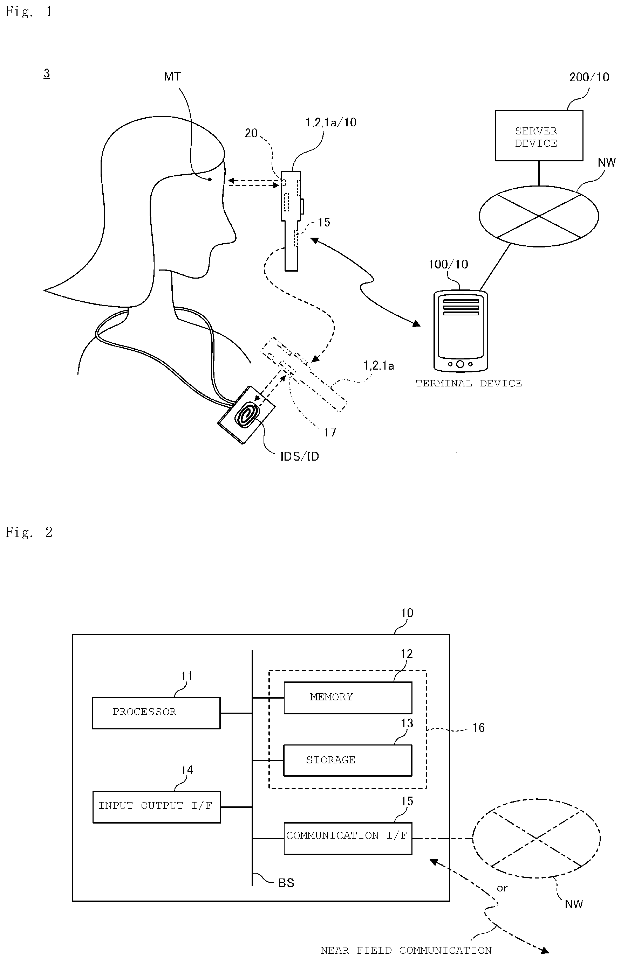

Fig. 1 is a constitutional view illustrating an example of the measuring device 1 and the measuring system 3 that includes the measuring device 1 according to the embodiment 1. As illustrated in Fig. 1, the measuring system 3 includes the measuring device 1, a terminal device 100 that is connected to the measuring device 1 by communication via a communication I/F 15 (described later), and a server device 200 that is connected to the terminal device 100 via network NW.

In the example illustrated in Fig. 1, the measuring device 1 is a body thermometer 1a, a measured object MT is a human and an amount to be measured by the measuring device 1 is a body temperature of a human. Further, the terminal device 100 is a so-called smartphone, and the server device 200 is a so-called cloud storage server.

The thermometer 1a measures a body temperature of the measured object MT by a measuring unit 20, reads identification information ID from an NFC tag (identification information source IDS) mounted on a company employee card by an ID reading unit 17, assigns (associates) identification code IC based on the identification information ID to the measured data MD, and records and stores this information as a record by a predetermined technique described later. The record stored in the thermometer 1a can be transmitted to other devices and hence, the record can be also held by the terminal device 100 and the server device 200 (the detail being described in embodiment 3).

The measuring device 1, the terminal device 100 and the server device 200 have substantially the same configuration and functions and hence, all these devices can be also referred to as the information processing device 10 respectively. Fig. 2 is a block diagram illustrating one example of the hardware configuration of the information processing device 10 used in the embodiments 1 to 3. As illustrated in Fig. 2, the information processing device 10 includes: a processor 11; a storage unit 16 constituted of a memory 12, a storage 13 and the like; an input/output I/F (Interface) 14 (a concept that includes a measuring unit 20, the ID reading unit 17 and the like described later), and the communication I/F (Interface) 15. These constitutional elements are connected to a bus BS.

### 2. Configuration of measuring device 1 according to the embodiment 1

Next, the detailed configuration of the measuring device 1 according to the embodiment 1 is described.

### (1) Fundamental function of measuring device 1

As described above, the measuring device 1 is a device for measuring an "amount to be measured" that is an amount representing a state or a property of the measured object MT. In this specification, either one of an "amount representing a state or a property of a measured object" and an "amount to be measured" can be used.

### (2) Functional configuration of measuring device 1

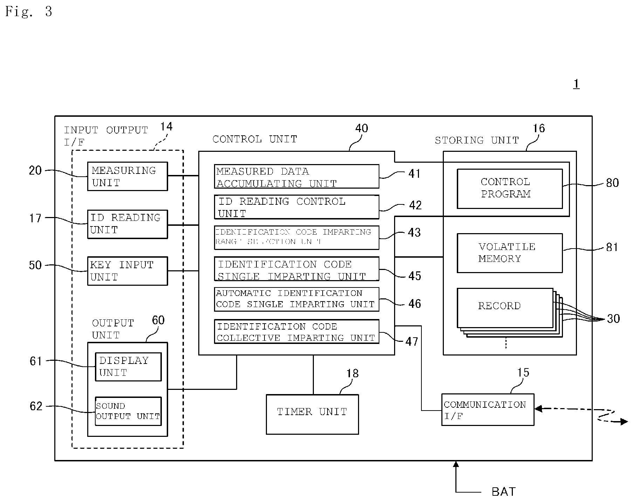

Fig. 3 is a block diagram illustrating one example of the functional configuration of the measuring device 1 according to the embodiment 1. As illustrated in Fig. 3, the measuring device 1 includes the measuring unit 20, the ID reading unit 17, the storage unit 16 and the control unit 40.

(2-1) The measuring unit 20 is a unit that measures a predetermined amount to be measured. As an example of an "amount to be measured", in a case in which the measured object MT is a living body (for example, human), a body temperature, a blood pressure, a heart rate, a blood sugar level, a weight, a body fat percentage, alcohol concentration contained in an exhaled breath and the like can be named. In a case in which the measured object MT is a substance, a temperature or a moisture in a room, concentration of carbon dioxide, a surface temperature of the substance, a weight of the substance, a sugar content of a food, a pressure and flow rate of a fluid and the like can be named. A physical quantity that expresses a so-called physical property and an industrial quantity that is a quantity useful in industry are also included in the amount to be measured. Further, "voltage value" that a sensor outputs as a result of sensing is also can be treated as the "amount to be measured" in this embodiment. The above-mentioned quantities are described exclusively for an exemplifying purpose, any quantity that can be measured by the measuring unit 20 can be treated as the "amount to be measured" in this embodiment.

As the measuring unit 20, for example, a temperature sensor, a blood pressure sensor, a pulse rate sensor, a pulse oximeter, a refractometer, a weight scale, a body fat meter, an alcohol detection sensor, a thermometer (measuring an ambient temperature), a hygrometer, a weight meter, a solar radiation meter, an illuminance meter, a pressure meter, an atmospheric pressure meter, a flow rate meter, a distance meter are included. The measuring unit 20 may be incorporated in the measuring device 1, or may be externally provided to the measuring device 1.

The measuring unit 20, after obtaining a measured data MD (not illustrated in the drawing) by measuring an amount to be measured, outputs the measured data MD to an inner memory of the processor 11 that constitutes the control unit 40, a volatile memory 81 of the storage unit 16 and the like via the bus BS (see also Fig. 2)

(2-2) The ID reading unit 17 reads identification information ID from the outside of the measuring device 1, and outputs the read identification information ID as an identification code IC. "identification code IC" is a unique code based on identification information ID, and is a code that can be processed by the control unit 40 described later.

As the identification information ID disposed on the outside of the measuring device 1, for example, a code stored in an electronic tag, a code converted into a one-dimensional barcode or a two-dimensional barcode, a code described in an identification code allocation table such as fingerprints, faces, irises, voiceprints and the like unique to respective individuals can be named. In this embodiment, "electronic tag" indicates a tag that conforms to an NFC standard, a tag based on RFID or the like that is configured such that identification information is readable by short distance radio communication.

As the ID reading unit 17, for example, an electronic tag reader, a barcode reader, a biometric authentication unit based on fingerprints or the like, a tenkey switch by which an identification code is manually inputted and the like can be adopted.

In a case in which the identification information ID is originally stored in an identification information source IDS in a state of an identification code IC, the ID reading unit 17 outputs the read identification information ID as an identification code IC in its original form. In a case in which the identification information ID is a biometric verification such as a fingerprint, the ID reading unit 17 converts the read identification information ID into an identification code IC that is in a form instructing the control unit 40 to process, and outputs the converted identification code IC.

The ID reading unit 17 outputs such an identification code IC to an inner memory of the processor 11, a volatile memory 81 of the storage unit 16 and the like via the bus BS (also see Fig. 2).

(2-3) The storage unit 16 is configured to store at least a plurality of records 30. As the storage unit 16, a rewritable device such as a RAM, an HDD, an SDD can be suitably adopted (see Fig. 2 together with Fig. 3). Further, the storage unit 16 includes a volatile memory 81 that temporarily stores data. Although not illustrated in the drawing, the storage unit 16 in this embodiment also includes an inner memory of the processor 11. Further, in the storage unit 16, a control program 80 that forms one of constitutional elements at the time of realizing the control unit 40 is stored. The record 30 is a unit of data in which measured data MD, identification code IC and the like can be written (the detail being described later).

(2-4) The control unit 40 is a unit that controls an operation of the measuring device 1. The control unit 40 may be constituted of either one of a dedicated hardware or a general-use hardware. With respect to the control unit 40 constituted of the general-use hardware, the control unit 40 may be constituted of the processor 11, the hardware such as the memory 12, and the control program 80 stored in the memory 12 or the control program 80 loaded to the memory 12 from the storage 13 or an external memory device not illustrated in the drawing as elements thereof (also see Fig. 2). In such a configuration, the control unit 40 can be realized by executing based on the control program 80 using the above-mentioned hardware resources. The detail of the control unit 40 is described later.

### (3) Other peripheral functional units

Besides the above-mentioned functional units, the measuring device 1 includes an input/output I/F 14, a timer unit 18, and a communication I/F 15.

The input/output I/F 14 may include, besides the above-mentioned measuring unit 20 and the ID reading unit 17, a key input unit 50, and an output unit 60 such as a display unit 61, a sound output unit 62 and the like. The key input unit 50 may be constituted of an input device such as a button switch, a dial switch or a key of a touch panel or the like, for example. These input devices receive operator's instructions such as starting of measurement, starting of ID reading, the selection of various modes, the confirmation of a selected content and the like from the operator of the measuring device 1. On the other hand, the display unit 61 may be constituted of a device such as an LCD or an LED so as to display predetermined information to the outside. The sound output unit 62 may be constituted of a device such as a buzzer or a speaker, and outputs a predetermined sound information to the outside. In the input/output I/F 14 (excluding the communication I/F), the input/output device itself, an input/output I/F circuit for communication with the input/output device, an input/output I/F program and the like are included. The processor 11 controls the input/output device via the input/output I/F circuit or the like.

The timer unit 18 outputs a point of time, an elapsed time and the like in response to a signal from the outside. The timer unit 18 may be constituted of a real-time clock (RTC) or the like, for example.

The communication I/F 15 is an I/F (interface) part that performs the communication between the measuring device 1 and an external equipment. For example, the communication I/F 15 may include a Bluetooth low energy (BLE)communication function, for example.

### (4) Data structure of record 30

Next, prior to the detailed description of the control unit 40, the data structure of the record 30 that the control unit 40 treats is described.

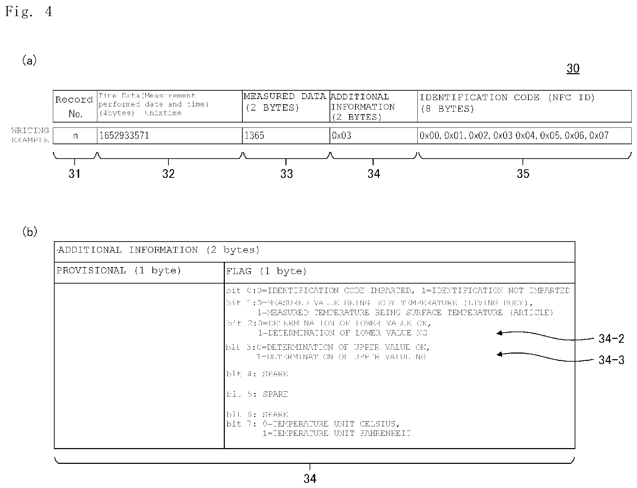

Fig. 4 is a view illustrating the data structure of the record 30 according to the embodiment 1. A first row in Fig. 4(a) indicates one example of a specification of respective fields that constitute the record 30, and a second row in the drawing indicates an example of data writing to the record 30.

In such a data structure, "record 30" means, as illustrated in Fig. 4(a) means, a unit of the data structure that at least includes a measured data field 33 in which measured data MD obtained from the single measurement by the measuring unit 20 is storable, and an identification code field 35 in which an identification code IC associated with the measured data MD is storable.

The record 30 may include, besides the above-mentioned fields, a record number filed 31, a time data field 32 in which time data is storable, an additional information field 34 in which additional information associated with the measured data is storable and the like. The number of bytes filled out in a parenthesis in the drawing is the number of bytes allocated to each field. However, the number of bytes exemplify an example, and can be suitably designed and changed.

"Time data" includes measurement date and time data (date, time) that has significance of a time stamp, and elapsed time data at the time of measurement counted from a reference time and the like.

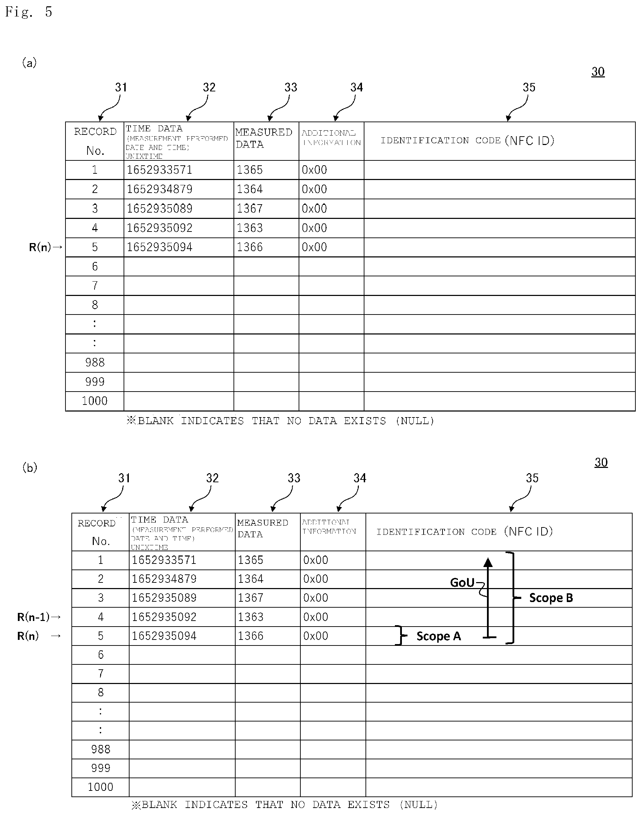

In the description of the embodiment 1, it is assumed that the record 30 is realized by a fixed table. In an example illustrated in Fig. 5 to 7, a memory frame in which 1000 pieces of records associated with measurements performed 1000 times are storable is prepared as a fixed table. Further, in this specification, there is a case in which a single record or a plurality of records are indicated by "symbol 30", and also a case in which a record that corresponds to a specified record number is indicated as "R (n)" (n being a subscript corresponding to the record number).

Fig. 4(b) illustrates an example of the detailed specification with respect to the additional information field 34 that forms a part of the record 30. In the additional information field 34, 1 byte is secured as a region of flags. Further, as the contents of 1 byte for such flags, various flags having significance illustrated in the drawing are allocated 1 bit by 1 bit. For example, with respect to the bit 2, the flag definition is made such that, when the measured data MD (measurement value) exceeds a lower limit value in terms of management, the flag is set to 1, and when the measured data MD does not exceed the lower limit value in terms of management, the flag is set to 0 (see symbol 34₋₂). With respect to the bit 3, the flag definition is made such that, when the measured data MD (measurement value) exceeds an upper limit value in terms of management, the flag is set to 1, and when the measured data MD does not exceed the upper limit value in terms of management, the flag is set to 0 (see symbol 34₋₃).

### (5) Detail of control unit 40

Next, the detail of the control unit 40 is described with reference to Fig. 3 to Fig. 7.

The control unit 40 includes a measured data accumulation unit 41, an ID reading control unit 42, an identification code assignment range selection unit 43, an identification code individual assignment unit 45, and an identification code bulk assignment unit 47 (see Fig. 3).

Fig. 5 to Fig. 7 are views illustrating an operation of the control unit 40, and are also views illustrating one example of a state in which data is written in "table" that constitutes the plurality of records 30. The blank column indicates a state (= NULL) in which no data is written in the blank column.

(5-1) The measured data accumulation unit 41 instructs the measuring unit 20 to measure an "amount to be measured (a body temperature or the like)" in accordance with an instruction from an operator, and has a function of writing the measured data MD obtained from such a single measurement to a predetermined field of the storage unit 16 as a part of the record 30.

In the above-mentioned operation, "an instruction of an operator" means that the operator presses a measurement button switch 51 in the above-mentioned example. However, the present invention is not limited to such a case, and, for example, "an instruction of an operator" means an instruction in general that makes the measuring unit 20 start the measurement such as an instruction using a voice or a gesture, for example.

For example, when the operator issues an instruction to start the measurement by pressing the measurement button switch 51 (described later) while directing the place where the measuring unit 20 (a temperature sensor in this embodiment) is accommodated toward the measured object MT, the measured data accumulation unit 41 makes the measuring unit 20 start sensing in response to such an instruction, and obtains measured data MD.

In this specification, "measured data MD obtained by the measurement of every one cycle", "data obtained from single measurement" and the like imply the measured data obtained by a single sensing, typically. However, the measured data is not limited to such measured data. The measured data includes measured data that is eventually outputted by averaging a plurality of sensing values obtained by sensing performed over multiple cycles, the measured data in a case in which a sensing value obtained by sensing in the last cycle out of a plurality of sensing is outputted as the measured data, and the like.

The control unit 40, when the measurement is performed by the measuring unit 20, may display the measured data MD relating to the measurement cycle on the display unit 61.

The measured data accumulation unit 41 performs a set (a series of processing) where (i) the measuring unit 20 is made to measure an amount to be measured in accordance with an instruction of an operator, and (ii) the measured data MD obtained by the measurement is written as a part of the record 30 of the storage unit 16 at least one or more times, and the records 30 in which the measured data MD is written are accumulated on the storage unit 16 one after another. Fig. 5(a) illustrates one example of a state of the table immediately after the measured data accumulation unit 41 is operated relating to the fifth measurement (record No. 5) is operated. In this embodiment, in the respective records R(1) to R(5) that correspond to the record numbers 1 to 5, the measured data MD is written in respective measured data fields 33. Further, along with such an operation, measurement date and time are written in the respective time data fields 32, and 0×00 is written in the respective additional information fields 34.

(5-2) The ID reading control unit 42 has a function of making the ID reading unit 17 read identification information ID.

An operator, for issuing an instruction to start ID reading, presses an ID reading button switch 52 (described later) while placing an identification information source IDS (an employee card), for example in front of the ID reading unit 17 (NFC tag reader 17a).

The control unit 40 is performing polling whether or not any one of button switches is pressed. In a case in which the pressed button switch is the ID reading button switch 52 (described later), the ID reading control unit 42 makes the ID reading unit 17 start reading of identification information ID using the above-mentioned instruction as a trigger. The ID reading unit 17 outputs (writes) the identification code IC based on the identification information ID to an inner memory of the processor 11, a volatile memory 81 of the storage unit 16 and the like.

(5-3) The identification code assignment range selection unit 43 has a function of prompting an operator to select an identification code assignment range that indicates a range of measured data MD (written measured data) to which a new identification code IC that is just obtained by reading is to be added.

To be more specific, when the identification code assignment range selection unit 43 obtains the new identification code IC in the manner as described above in (5-2), the identification code assignment range selection unit 43 references the storage unit 16, and prompts an operator to select "(A) to assign an identification code to a single measured data" or "(B) to assign an identification code to a plurality of measured data" when the storage unit 16 is in a state in which the identification code IC is not written (being null) in the identification code field 35 of the record R(n-1) that is one cycle prior to the record R(n) that corresponds to the most recent measurement cycle.

In prompting the operator to select either "(A) to assign an identification code to a single measured data or "(B) to assign an identification code to a plurality of measured data, such the selection may be performed in accordance with the guidance of the output unit 60 (display part 61, sound output part 62 or the like) in a state in which measured data is displayed on the display part 61.

The selection of the operation mode can be guided while displaying the measurement data MD on the display unit 61 and hence, the occurrence of an error in the operation can be reduced.

In the above-mentioned operations, as a condition for starting the referencing of the storage unit 16, outputting (writing) of the identification code IC from the ID reading unit 17 may be used, or the notification from the ID reading unit 17 that the ID has read the ID may be used instead of the above-mentioned outputting of the identification code IC. Further, "most recent measurement cycle" means the measurement cycle at which the measurement is performed most recently (the measurement is performed most recently).

Further, "when a state is such that the identification code IC is not written in the identification code field 35 of the record R(n-1) that corresponds to one cycle prior to the record R(n), which corresponds to the most recent measurement cycle" can be also expressed as "in a case in which it is determined that the records in which no identification code IC is written consecutively exist including the record R(n) of the most recent measurement cycle when the records that correspond to the past measurement cycles are looked back time-sequentially in a retroactive manner from the record R(n) that corresponds to most recent measurement cycle.

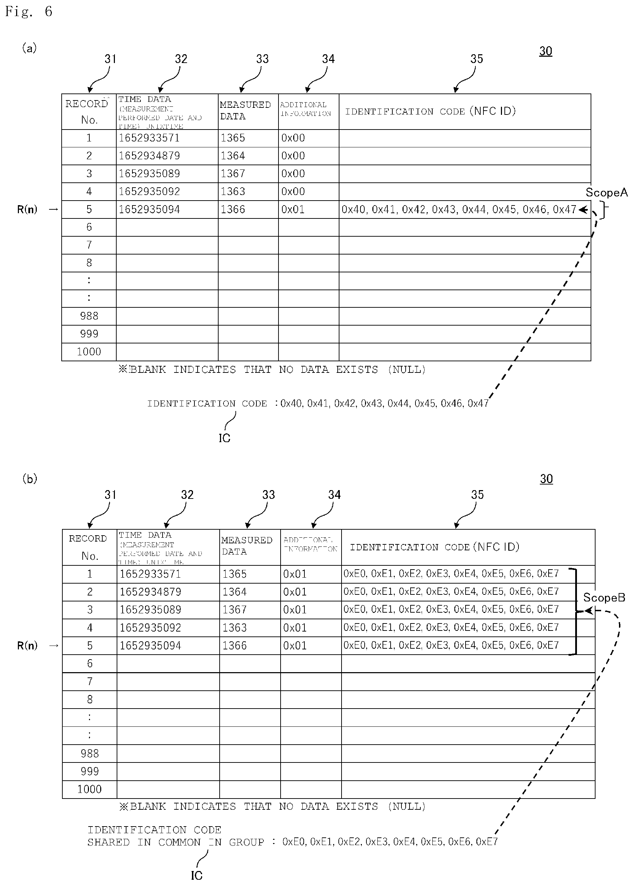

Hereinafter, the description is continued by exemplifying specific examples. Fig. 5(a) is a view illustrating a state of a table immediately after the measured data accumulating unit 41 is operated, and Fig. 5(b) is a view illustrating choices within a range in which identification codes IC are assigned. Fig. 6(a) is a view illustrating a mode of the table when the identification code individual assignment unit is operated, and Fig. 6(b) is a view illustrating the mode of the table when the identification code bulk assignment unit is operated.

For example, at a timing that a new identification code IC is obtained, when the state in the storage unit 16 is a state illustrated in Fig. 5(a), the record that corresponds to the most recent measurement cycle becomes a record having a record number 5 indicated by R(n). At this stage of processing, with respect to the record R(n-1) that is one cycle prior to the record R(n), the storage unit 16 is in a state in which the identification code IC is not written in the identification code field 35.

In such a state, identification code assignment range selection unit 43 performs the following operation. That is, the identification code assignment range selection unit 43 gives an inquiry to an operator whether the operator performs (A) to assign an identification code to single measured data (a range indicated by a scope A in Fig. 5(b) )or (B) to assign an identification code to a plurality of measurement data (a range indicated by a scope B in Fig. 5(b)) by displaying such an inquiry on the display unit 61, and prompts the operator to select either one of the ranges using a key input device of a key input unit 50, for example.

(5-4) In a case where the operator selects (A) to assign the identification code to the single measured data, that is, selects the scope A, the identification code individual assignment unit 45, as illustrated in Fig. 6(a), writes the newly obtained identification code IC to the record R(n) that corresponds to the most recent measurement cycle. Specifically, the identification code individual assignment unit 45 writes the identification code IC to the identification code field 35 of the record R(n). With such an operation, it is possible to assign the identification code IC only to the most recent measured data (making the identification code IC associated with the most recent measured data).

(5-5) The identification code bulk assignment unit 47 writes the identification code IC. That is, in a case where the operator selects (B) to assign an identification code to a plurality of measured data, that is, selects the scope B, as viewed retroactively in order from the record R(n) of the most recent measurement cycle, the identification code bulk assignment unit 47 collectively writes the newly obtained same identification codes IC to each consecutive record in a state in which no identification code IC is written in the identification code field 35 (the respective records corresponding to the record numbers 1 to 5 in the example illustrated in Fig. 5).

Accordingly, as described in Fig. 6(b), from the record R(n) (the record corresponding to the record number 5) to the retroactive record corresponding to the record number 1, the same identification code IC shared in common by the group is written in the identification code fields 35 of the respective records. In this manner, it is possible to assign (associate) the same identification code IC shared in common by the group to the plurality of measured data retroactive from measured data of the most recent measurement cycle including the data of the most recent measurement cycle.

(5-6) The control unit 40 includes "automatic identification code individual assignment unit 46".

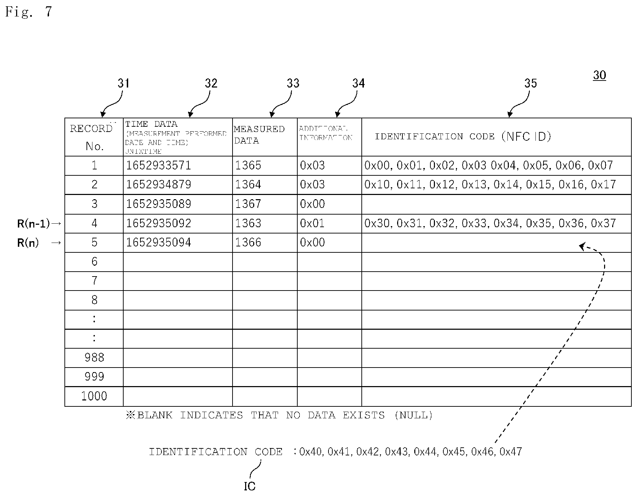

The automatic identification code individual assignment unit 46 automatically writes the identification code IC as follows. More specifically, when the ID acquisition unit newly obtains the identification code IC by reading identification information ID, the identification code individual assignment unit 46 references the storage unit 16, and when the storage unit 16 is in a state in which the identification code IC is written in the identification code field 35 of the record R(n-1) that is one cycle prior to the record R(n) that corresponds to the most recent measurement cycle (for example, when the storage unit 16 is in a state illustrated in Fig. 7), the identification code individual assignment unit 46 automatically writes the newly obtained identification code IC to the record R(n) that corresponds to the most recent measurement cycle.

In other words, as viewed retroactively from the most recent cycle, the identification code IC is assigned to the record R(n-1) that is one cycle prior to the record R(n), while in a case where the identification code IC is not yet assigned only to the record R(n) of the most recent measurement cycle, the identification code IC is automatically written.

### (5-7) Time stamp

The control unit 40 may be configured to write, as a part of the record R(n), together with writing of the measured data MD, time data (not illustrated in the drawing) that the timer unit 18 outputs, to a time data field 32 in an associated manner with the measured data MD (see Fig. 5 to Fig. 7). By referencing the time data on an external device side such as a terminal device 100, at the time of processing the measured data MD, it is possible to prevent overwriting, missing of processing and the like of data with certainty.

### 3. Advantageous effects of measuring device 1 according to embodiment 1

(1) In the measuring device 1 according to the embodiment 1, the measured data accumulating unit 41 instructs the measuring unit 20 to measure an amount to be measured in response to an instruction from an operator and, at the same time, instructs the measurement unit 20 to write the measured data MD obtained by the measurement as a part of the record R(n) in the storage unit 16 at least one or more times.

That is, even when an operation of ID reading is not performed, first of all, the measurement and accumulation of measured data can be performed one after another.

Accordingly, from a viewpoint of a user who mainly wants to use simply an operation mode in which the temperature measurement (and the accumulation of measured data) is only performed, as an operation, the user performs only one action of instructing the measurement (for example, only pressing the measurement button switch 51). In this manner, according to the measuring device 1 of the embodiment 1, it is possible to provide a measuring device having the simple operation specification as an existing general-use thermometer or body thermometer without prompting the user to be conscious of the presence of the other operation modes such as assigning an identification code.

Further, when the ID acquisition unit newly obtains an identification code by reading identification information, the identification code assignment range selection unit 43 references the storage unit, and when the storage unit is in a state in which the identification code is not written in the identification code field of the record that is one cycle prior to the record that corresponds to the most recent measurement cycle, prompts the operator to select (A) to assign the an identification code to single measured data or (B) to assign an identification code to a plurality of measured data. Then, in a case in which the operator selects (A), the identification code unit writes the newly obtained identification code to only the record that corresponds to most recent time, while in a case in which the operator selects (B), the identification code bulk assignment unit writes the same identification code collectively to a predetermined plurality of records including the record of the most recent measurement cycle.

In this manner, according to the measuring device 1, it is possible to operate the measuring device 1 in a mode in which the measuring device 1 simply performs the temperature measurement (and the accumulation of measured data). It is also possible to dynamically shift the mode of operation of the measuring device 1 to another mode of operation (a mode of operation where the identification code IC assigned later) by reading ID of an identification information source IDS presented by pressing an ID reading button even in the midst of the simple temperature measurement mode. In this manner, the measuring device 1 can be used in various operation modes by dynamically switching the mode. As a result, the measurement device 1 can realize both the assurance of the convenient operability and the increase of selection choices of operation modes (only simple measurement/assigning identification code).

Further, the identification code bulk assignment unit is configured to collectively write the same identification code to all each consecutive record that is in a state in which no identification code is written in the identification code field. Accordingly, unlike the measuring devices described in patent literatures 1 and 2, it is unnecessary to measure, to read identification information and to assign of an identification code based on the identification information for each single measurement as a set every time, and the identification code can be collectively assigned by operating identification code bulk assignment once. Also, from this point of view, the measuring device 1 according to the embodiment 1 has the highly convenient operability.

In a case in which it is necessary to assign the identification codes IC collectively with respect to a plurality of measured data after a certain point of time of measurement in the midst of a mode of the single temperature measurement, for example, the identification code IC is read in advance with respect to the measured data at the certain point of time of the measurement, and the identification code IC is written in the identification code field 35 of the record 30 thus realizing the bulk assignment of the identification codes IC. Alternatively, during recording at the measurement point of time, the measuring device may write other separator code (not illustrated in the drawing) that can recognize the starting point of time of a plurality of measured data via the input/output I/F 14. Further, such separator codes may be written in regions other than the identification code field 35 of the record 30 such that the measuring device can recognize the zoning codes.

Further, according to the measuring device 1, it is also easy to assign plural kinds of single identification codes IC. For example, first, measured data is accumulated and one identification code IC is assigned using one identification information ID and, subsequently, measured data is accumulated again, and another identification code IC can be assigned using another identification information ID. Accordingly, it is possible to easily perform the measured data management having general-use property and flexibility in which an identification code is assigned constantly to a group of arbitrary objects to be measured.

As can be understood from above, according to the embodiment 1, it is possible to provide the measuring device 1 that can realize various operation modes without impairing the ease of the operation and, at the same time, can realize assigning the identification code with respect to the single or the plurality of measured data in the form that the measuring device 1 has general-use property.

(2) The measuring device 1 according to the embodiment 1 includes "automatic identification code individual assignment unit 46". Accordingly, in a predetermined case, an operator does not receive an inquiry about an assignment range of the identification code whether assigning the identification code is for (A) single measured data or (B) a plurality of measured data and hence, the operation becomes more convenient.

### 4. Configuration and advantageous effects obtained by method of controlling a measuring device 1.

The method of controlling a measuring device 1 according to the embodiment 1 is a method of controlling a measuring device 1 that measures an amount (an amount to be measured) that represents a state or a property of a measured object. The measuring device 1 includes the measuring unit 20, the ID reading unit 17, the storage unit 16, and the control unit 40. The configuration of the measuring device 1 used in the method is substantially equal to the configuration of the measuring device 1 described heretofore and hence, the above-mentioned description is also used in this method of controlling the measuring device 1.

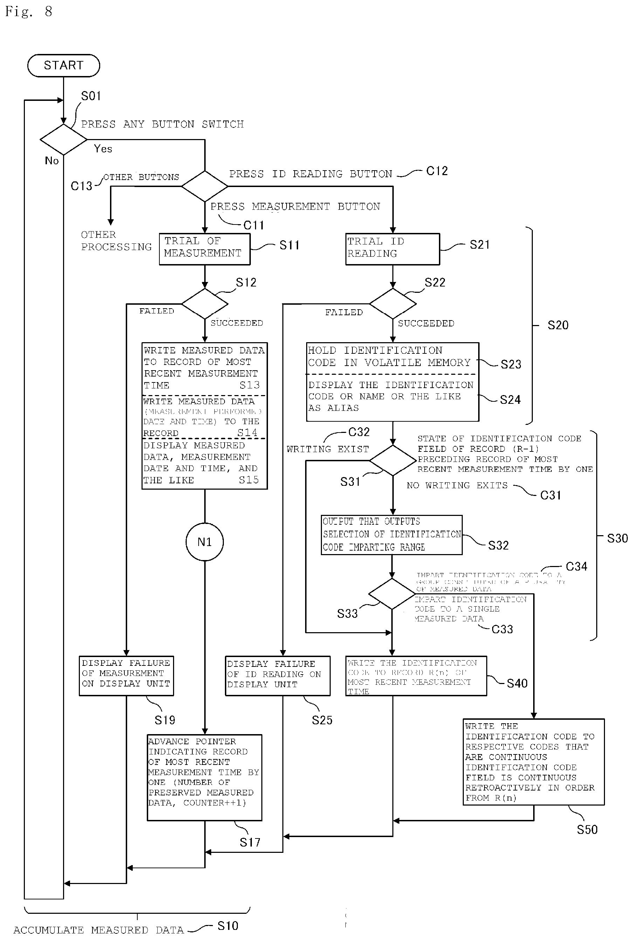

Fig. 8 is a flowchart illustrating one example of the method of controlling the measuring device 1 according to the embodiment 1. As illustrated in Fig. 8, the method of controlling the measuring device 1 includes at least a measured data accumulating step S10, an identification code assignment range selecting step S30, an identification code individual assigning step S40, and an identification code bulk assigning step S50. In addition to these steps, the method of controlling the measuring device 1 may include an ID reading control step S20.

(1) The measured data accumulating step S10 includes at least a measuring step (C11, S11, S12) and a measured data writing step (S13).

In the measuring step, in a case the control unit 40 recognizes that a certain button is pressed from a standby state in which polling is being performed whether a certain button switch is pressed or not (S01), the control unit 40 instructs the measuring unit 20 to measure an amount to be measured in response to an instruction (C11) of an operator (S11, S12). In the measured data writing step S13, measured data MD that is obtained by measuring one cycle is written as a part of the record R(n) in the storage unit 16. In the measured data accumulating step S10, the set of the measuring step and the measured data writing step S13 is performed one or more times, for example. Such a set of steps is repeatedly performed while advancing a pointer indicating the record of most recent measurement cycle, for example (S17).

At the substantially same stage as the measured data writing step S13, time data is written to the record R(n) (S14), or measured data MD and time data such as measurement date and time may be displayed on the display unit 61 (S15).

(2) In the identification code assignment range selecting step S30, when the ID reading unit 17 obtains the identification code IC by newly reading the identification information ID (C12, S21, S22, S23), the control unit 40 references the storage unit 16 (S31), and prompts an operator to select either (A) to assign the ID code to the single measured data (S33, C33) or (B) to assign the identification code to the plurality of measured data (S33, C34) when the storage unit 16 is in a state in which the identification code IC is not written in the identification code field 35 of the record R(n-1) that is one cycle prior to the record R(n) corresponding to the most recent measurement cycle. (see also Fig. 5)

On the other hand, when the identification code IC is written in the identification code field 35 of the record R(n-1) that is one cycle prior to the record R(n) corresponding to the most recent measurement cycle in the storage unit 16 (C32), the control unit 40 automatically performs the identification code individual assigning step S40 without making an inquiry to the operator (corresponding to the automatic identification code individual assignment unit 46 described previously).

At the stage substantially equal to the acquisition of the identification code IC (S23), the identification code IC may be displayed on the display unit 61 (S24). Further, when the identification code IC (employee number or the like) is firstly obtained, the identification code IC is transmitted to the terminal device 100, and in the terminal device 100, the registration management of information relating to the new identification code IC may be performed by naming such as a name as an alias, and when the same identification code IC is obtained in the next time and thereafter, at the stage substantially equal to the acquisition of the identification code IC (S23), the display of a name or the like as alias is displayed on the display unit 61.

(3) In the identification code individual assigning step S40, when the operator selects (A) to assign the identification code to the single measured data (C33) or when the identification code IC is written in the identification code field 35 of the record R(n-1) one cycle prior to the record R(n) that corresponds to the most recent measurement cycle of the storage unit 16 (C32), the control unit 40 writes the newly obtained identification code IC only to the record R(n) that corresponds to the recent measurement cycle (see also Fig. 6(a)).

(4) In the identification code bulk assigning step S50, when the operator selects (B) to assign the identification code to a plurality of measured data (C34), the control unit 40 collectively writes the newly obtained same identification code IC to each consecutive record in which no identification code IC is written in the identification code field 35 as viewed retroactively in order from the record R(n) of the most recent measurement cycle (also see Fig. 6(b)).

Then, the control unit 40 waits whether or not any button switch is pressed again. When the measurement button switch 51 is pressed, the measuring step (C11, S11, S12) and the measured data writing step (S13) are performed. Further, when the ID reading unit 17 obtains the identification code IC by newly reading the identification information ID (C12, S21, S22, S23 and S20 that includes these steps), the control unit 40 performs the identification code assignment range selecting step S30, the identification code individual assigning step S40, the identification code bulk assigning step S50 and the like in accordance with the substantially same processing as the previous cycle.

The measured data accumulating step S10, the ID reading control step S20, the identification code assignment range selecting step S30, the identification code individual assigning step S40 and the identification code bulk assigning step S50 have substantially the same characteristics as the characteristics that the above-mentioned measured data accumulating unit 41, the ID reading control unit 42, the identification code assignment range selection unit 43, the identification code individual assignment unit 45 and the identification code bulk assignment unit 47 have. Accordingly, the description of these units described above are also used similarly in this step, and the repeated detailed explanation of these units is omitted. Further, the above-mentioned automatic identification code assignment unit 46 corresponds to the condition branching S31, the condition branching C32 and the identification code individual assigning step S40 in the flowchart illustrated in Fig. 8.

The method of controlling the measuring device 1 according to the embodiment 1 can be carried out using the measuring device 1 according to the embodiment 1 and hence, the method of controlling the measuring device 1 according to the embodiment 1 can have the advantageous effects based on the technical features of the measuring device 1 in the same manner.

### 5. Example of measuring device 1

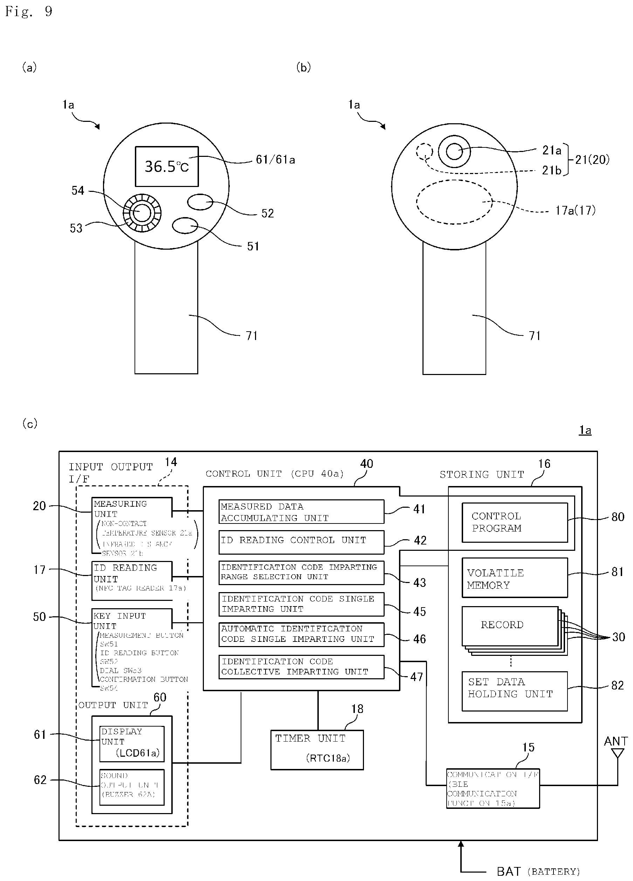

Fig. 9 is a view illustrating a measuring device 1a according to an example. Fig. 9(a) is a front view of the measuring device 1a, and Fig. 9(b) is a back view of the measuring device 1a.

The measuring device 1a according to the example (hereinafter, also referred to as "body thermometer 1a") is a body thermometer that measures a body thermometer of a human where the human is a measured object MT.

As illustrated in Fig. 9(a), on a front surface of the body thermometer 1a, as key input units 50, a measurement button switch 51, an ID reading button switch 52, a dial switch 53, and a confirmation button switch 54 are disposed. The control unit, using this dial switch 53, may select (A) to assign the identification code to the single measured data and (B) to assign the identification code to the plurality of measured data. Further, an LCD 61a is disposed on a front surface of the body thermometer 1a as the display unit 61. Symbol 71 indicates a grip portion.

As illustrated in Fig. 9(b), a non-contact temperature sensor 21a is disposed on a back surface of the body thermometer 1a. Also, on the back surface of the body thermometer 1a, an infrared distance sensor 21b is disposed together with the non-contact temperature sensor 21a. In measuring a body temperature of a human, in a case in which a distance between a surface of a skin of the human and the non-contact temperature sensor 21a is inappropriate, there may be a case in which the body temperature measurement cannot be properly performed. Accordingly, the body thermometer 1a is configured to be capable to properly measure the body temperature while measuring the distance by the infrared distance sensor 21b. Further, on a back surface side of the body thermometer 1a, an NFC tag reader 17a that constitutes an ID reader 17 is disposed in a casing. In this specification, there may be a case in which a portion of the body thermometer 1a around an area to which an infrared light generated by the non-contact temperature sensor 21a is projected and received is referred to as "sensor head".

Fig. 9(c) is a block diagram illustrating one example of the functional configuration of the body thermometer 1a.

As illustrated in Fig. 9(c), in the body thermometer 1a, a CPU 40a that is a processor 11 constituting a part of the control unit 40; a RTC18a that is a timer unit 18 and a buzzer 62a that is a sound outputting unit 62 are respectively implemented. The entirety of the control unit 40 (measured data accumulating unit 41, the ID reading control unit 42, the identification code assignment range selection unit 43, the identification code individual assignment unit 45, the automatic identification code individual assignment unit 46 and the identification code bulk assignment unit 47) may be constituted of the CPU 40a. A setting data holding unit 82 is also provided in the storage unit 16. The setting data holding unit 82 holds data relating to various settings of the measuring device 1 (for example, the presence or non-presence of a beep sound by the buzzer 62a or the like). ABLE communication function 15a is implemented in the body thermometer 1a as a communication I/F 15, and an antenna ANT is connected to the BLE communication function 15a.

The identification information ID is stored in an electronic tag in accordance with Near Field Communication (NFC) standard. The ID reading unit 17 is configured to read identification information ID from the outside via a short distance wireless communication in accordance with the NFC standard. By adopting such a configuration, even when electronic tags having substantially the same configuration are positioned in a scattered manner around the ID reading unit 17, the ID reading unit 17 can properly read ID without causing interference.

The use example of the body thermometer 1a is already illustrated in Fig. 1 and is described in the column "(1) Summary of measuring system 3 in 1. Measuring device 1 and measuring system 3 that includes the measuring device 1".

### [Embodiment 2]

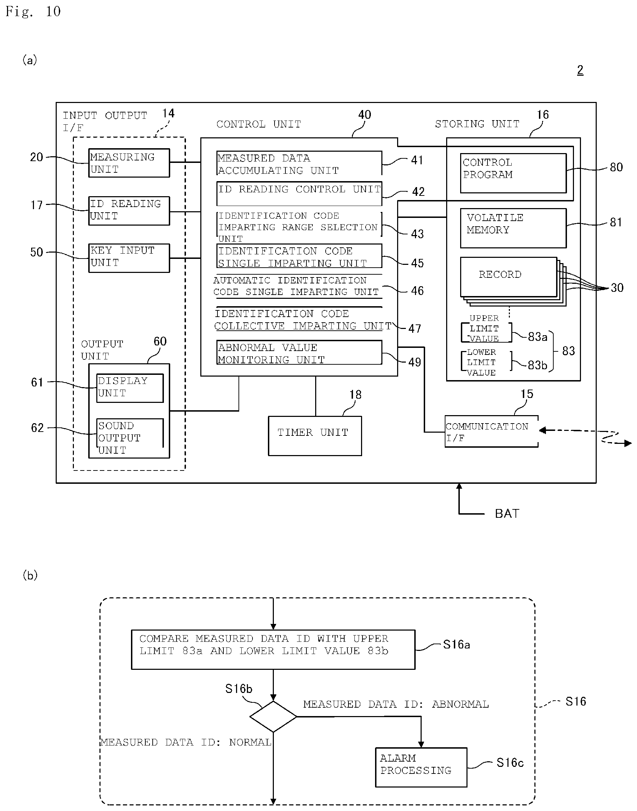

Fig. 10 is a view illustrating a measuring device 2 according to an embodiment 2. Fig. 10 (a) is a block diagram illustrating one example of the functional configuration of the measuring device 2. The measuring device 2 according to the embodiment 2 has basically substantially the same configuration as the measuring device 1 according to the embodiment 1. However, the measuring device 2 according to the embodiment 2 differs from the measuring device 1 according to the embodiment 1 with respect to a point that the measuring device 2 further includes abnormal value monitoring unit 49.

As illustrated in Fig. 10 (a), in the measuring device 2 according to the embodiment 2, a control unit 40 further includes the abnormal value monitoring unit 49. The abnormal value monitoring unit 49 may be configured such that, when measured data MD exceeds or falls below a predetermined reference value, "abnormal value flag (bit 3 or bit 2 in Fig. 4)" is set in an additional information field 34 of the record corresponding to the measured data MD. It is assumed that the additional information field 34 capable of storing additional information associated with the measured data MD is further included in the respective records (see Fig. 4).

Fig. 10 (b) is a flowchart obtained by taking out an abnormal value monitoring step S16. The flow of the abnormal value monitoring step S16 can be implemented (synthesized) in-line at a position indicated by N1 in the flowchart illustrated in Fig. 8.

In the abnormal value monitoring step S16, on a premise that an upper limit value 83a and a lower limit value 83b that form reference values 83 are preliminarily stored in the storage unit 16, first, measured data ID and the upper limit value 83a are compared to each other, and/or the measured data ID and the lower limit value 83b are compared to each to other (S16a). As a result, when the measured data MD exceeds the upper limit value 83a or the measured data MD falls lower than the lower limit value 83b, alarming processing S16c is performed. As the alarming processing S16c, the display or the generation of a sound that informs the occurrence of abnormality may be performed. Further, the alarming processing S16c also includes setting "abnormal value flag" in the additional information/field 34 of the record that corresponds to the measured data MD. The processing in the above-mentioned abnormal value monitoring step S16 is merely an example, and the processing is not limited to such processing.

The measuring device 2 includes the abnormal value monitoring unit 49, and has abnormal value monitoring step S16. Accordingly, an abnormal value flag can be set with respect to the measured data that exceeds or falls below the predetermined reference value (for example, a value of a body temperature that is considered as high fever). Accordingly, an analysis and tracking investigation or the like can be performed in detail later with respect to a population (specific singles, specific group, or the like) that is a measured object having an identification code on which an abnormal value flag is set. As a result, accurate inspection can be performed again with respect to such population. Accordingly, the measuring device 2 can be preferably used in the discovery, the identification or the like of a cluster in the prevention of an infectious disease, for example.

The description of the constitutional technical features in the abnormal value monitoring unit 49 and the description of the technical features in the abnormal value monitoring step S16 are mutually applicable between the abnormal value monitoring unit 49 and the abnormal value monitoring step S16. Further, the measuring device 2 according to the embodiment 2 has basically substantially the same constitution with the measuring device 1 according to the embodiment 1 with respect to the configurations other than the point that the measuring device 2 according to the embodiment 2 further includes the abnormal value monitoring unit 49. Accordingly, the measuring device 2 has substantially the same advantageous effects corresponding to the advantageous effects amongst all advantageous effects that the measuring device 1 has.

### [Embodiment 3]

Next, the description is made with respect to a measuring system 3 that includes the measuring device 1 according to the embodiment 1, the measuring device 2 according to the embodiment 2, and the body thermometer 1a and the like according to the examples (hereinafter expressed as "measuring device 1" representatively) as a part of constitutional elements. The summary of the measuring system 3 can be understood by also referencing Fig. 1 and the column [1. Measuring device 1 and measuring system 3 that includes measuring device 1].

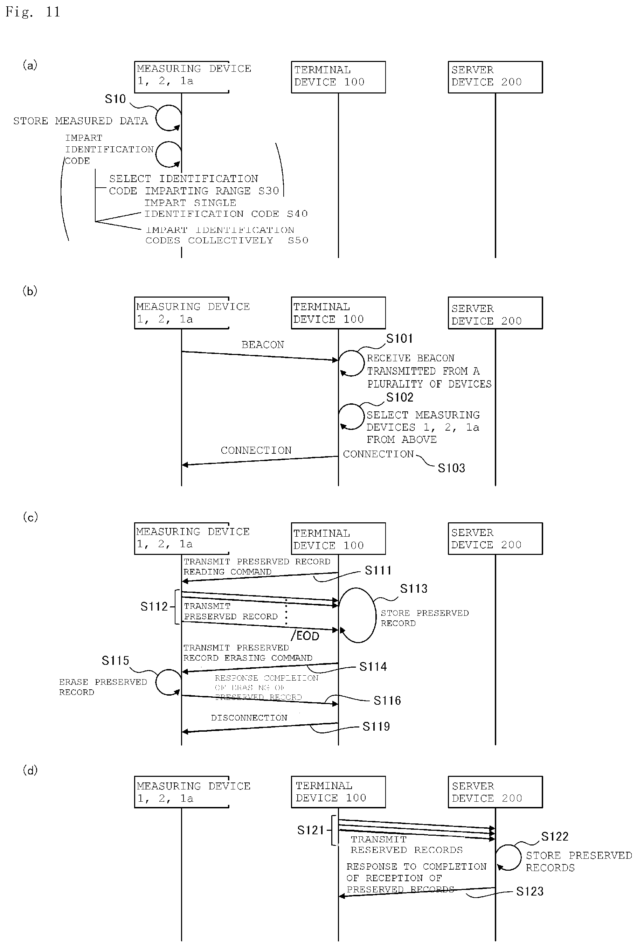

The measuring system 3 includes at least the measuring device 1 provided with the communication I/F 15, and the terminal device 100 that is connected to the measuring device 1 through communication via the communication I/F 15. The measuring device 1, by using a predetermined command as a trigger, transmits "stored record" that is the record in which measured data MD is written to the terminal device 100. The terminal device 100 receives the stored record transmitted from the measuring devices 1, 2, 1a. These sequences can be realized in accordance with steps illustrated in Fig. 11, for example.

Fig. 11 is an example of a sequence diagram illustrating an operation of the measuring system 3 according to the embodiment 3. Fig. 11 (a) is a sequence diagram of accumulating measured data and of assigning an identification code. The detail of the measured data accumulating step S10 and steps S30 to S50 of assigning identification code has been already described using Fig. 8. By performing these steps, the record (stored record) in which at least measured data MD is written is generated and stored. Fig. 11(b) is a sequence diagram illustrating the summary of establishing the connection between the terminal device 100 and the measuring device 1. The connection is established in accordance with the sequence described in the drawing so that the measuring device 1 and the terminal device 100 can communicate with each other. Fig. 11(c) is a sequence diagram illustrating an example of the transmission of the stored record from the measuring device 1 to the terminal device 100. Fig. 11 (d) is a sequence diagram illustrating an example of the transmission of the stored record from the terminal device 100 to the server device 200.

When the measuring device 1 receives a stored record reading command S111 from the terminal device 100, the control unit 40 of the measuring device 1 instructs the communication I/F 15 to transmit the record 30 stored in the storage unit 16 to the terminal device 100 that is the external device (S112). At this stage of processing, on a terminal device 100 side, the content of the record 30 is received, and the terminal device 100 stores the content of the record 30 in its own storage unit 16 (S113). Accordingly, it is possible to confirm and analyze the stored record also at the terminal device 100.

In such processing, even in a state in which one identification code is written in only the sole record (that is, an identification code ICa being recorded in the measured data MD on a one-to-one basis), and also in a state in which another identification code is written in common in a plurality of records (that is, an identification code ICb that differs from the identification code Ica being recorded in a plurality of measured data MD on a one-to-many basis), the control unit 40 may be configured to transmit these plurality of records collectively to the terminal device 100 that is the external device with respect to the communication I/F 15. With such a configuration, it is unnecessary to transmit every one of different kinds of data and hence, an operation becomes convenient whereby the operability can be enhanced.

Further, even in a case in which there exists a record 30 in which the identification code IC is not written, the control unit 40 may collectively transmit the records 30 together with the record in which the identification code IC is not written.

Further, the control unit 40 of the measuring device 1 may be configured such that, when the communication I/F 15 has transmitted all records 30 stored in the storage unit 16 (S112), the control unit 40 erases all records 30 that were stored in the storage unit 16 and were already transmitted. Alternatively, the control unit 40 of the measuring device 1 may be configured such that, after the terminal device 100 has received all records 30, the control unit 40 transmits, from the terminal device 100 to the measuring device 1, a command for erasing all records 30 that have been already transmitted from the measuring device 1.

In this manner, by erasing the records 30 that have been already transmitted, it is unnecessary for the control unit 40 to have a large memory for work and hence, a memory capacity of the entire storage unit 16 can be set small whereby it is possible to provide a power-saving miniaturized measuring device. Further, by erasing the records 30 that have been already transmitted, it is possible to prevent an erroneous operation such as the retransmission where data is transmitted in a duplicated manner.

The present invention has been described with reference to the above-mentioned embodiments heretofore. However, the present invention is not limited by the above-mentioned embodiments. The present invention can be carried out in various modes without departing from the gist of the present invention. For example, the following modifications are also conceivable.

(1) In the respective embodiments and examples, the identification code assignment range is selected when the storage unit 16 is in a state in which the identification code IC is not written in the identification code field 35 of the record R(n-1) that is one cycle prior to the record R (n) that corresponds to the most recent measurement cycle. However, the present invention is not limited to such a case. For example, the control unit 40 may be configured such that, when the state of the storage unit 16 is in such a state, the identification code IC is written automatically to all records where no data is written in the identification code field 35 (= NULL) without asking an operator.
(2) In the respective embodiments and examples, the description has been made with respect to the example where the fixed table capable of recording 1000 records as the data structure that can hold a plurality of records 30 for every measurement cycle is adopted. However, the present invention is not limited to such a configuration. For example, a structural body that uses the respective fields described in Fig. 4(a) as members is defined, and such "arrangement of the structural body" may be adopted as the data structure capable of holding a plurality of records 30 for every measurement cycle. In this case, "the record number member, the time data member, the measured data member, the additional information member and identification code member in the structural body" in the present invention may be applied to the present invention by alternatively reading these members as "the record number field 31, the time data field 32, the measurement data field 33, additional information field 34, and the identification code field 35". Further, for example, in the same manner as described above, "the list structure of the structural body" may be adopted.

### Reference signs list

1, 1a, 2: measuring device
1a: thermometer
3: measuring system
10: information processing device
11: processor
12: memory
13: storage
14: input output I/F
15: communication I/F
15a: BLE communication function
16: storage unit
17: ID reading unit
17a: NFC tag reader
18: timer unit
18a: RTC
20: measuring unit
21a: non-contact temperature sensor
21b: infrared distance sensor
30: record
31: record number field
32: time data field
33: measured data field
34: additional information field
35: identification code field
40: control unit
40a: CPU
41: measured data accumulating unit
42: ID reading control unit
43: identification code assignment range selection unit
45: identification code individual assignment unit
46: automatic identification code individual assignment unit
47: identification code bulk assignment unit
49: abnormal value monitoring unit
50: key input unit
51: measurement button switch
52: ID reading button switch
53: dial switch
54: confirmation button switch
60: output unit
61: display unit
61a: LCD
62: sound output unit
62a: buzzer
80: control program
81: volatile memory
82: setting data holding unit
83: reference value
83a: upper limit value
83b: lower limit value
100: terminal device
200: server device

## Claims

1. A measuring device for measuring an "amount to be measured" that is an amount indicating a state or a property of a measured object, the measuring device comprising:
a measuring unit configured to measure the amount to be measured;
an ID reading unit configured to read identification information from outside the measuring device, and configured to output the read identification information as an identification code;
a storage unit configured to be capable of storing a plurality of records, where a unit of a data structure, which includes at least: a measured data field capable of storing measured data obtained from a single measurement by the measurement unit; and an identification code field capable of storing the identification code associated with the measured data is defined as one "record"; and
a control unit configured to control an operation of the measuring device, wherein
the control unit includes:
a measured data accumulating unit configured to instruct the measuring unit to measure the amount to be measured in response to an operator's instruction, and, configured to write the measured data obtained by the measurement as a part of the record of the storage unit one or more times;
an identification code assignment range selection unit configured to reference the storage unit when the ID acquisition unit newly reads the identification information and obtains the identification code, and configured to, when the storage unit is in a state in which the identification code has not been written in an identification code field of the record that is one cycle prior to the record that corresponds to the most recent measurement cycle, prompt an operator to select either "(A) to assign an identification code to a single measured data" or "(B) to assign an identification code to a plurality of measured data"
an identification code individual assignment unit configured, when the operator selects "(A) to assign an identification code to a single measured data", to write the newly obtained identification code only to the record that corresponds to the most recent measurement cycle and
an identification code bulk assignment unit configured, when the operator selects "(B) to assign an identification code to a plurality of measured data", to collectively write the newly obtained same identification code to each consecutive record in which no identification code is written in the identification code field as viewed retroactively in order from the record of the most recent measurement cycle.

2. The measuring device according to claim 1, wherein
the control unit includes "an automatic identification code individual assignment unit" configured to reference the memory unit when the ID acquisition unit newly reads the identification information and obtains the identification code, and automatically writes the newly obtained identification code to the record that corresponds to the most recent measurement cycle when the storage unit is in a state in which the identification code is written in the identification code field of the record one cycle prior to the record that corresponds to the most recent measurement cycle.

3. The measuring device according to claim 1 or 2, further comprising a display unit, wherein
the control unit is configured to instruct the display unit to display the measured data relating to the measurement cycle to the display unit when the measurement by the measuring unit is performed, and
the control unit is configured to instruct an operator to, at the time of prompting the operator to select either "(A) to assign an identification code to a single measured data" or "(B) to assign an identification code to a plurality of measured data", to make the selection in accordance with guidance provided by an output unit, in a state in which the measured data is displayed on the display unit.

4. The measuring device according to any one of claims 1 to 3, further comprising a communication I/F, wherein
the control unit is configured to instruct the communication I/F to transmit the record stored in the storage unit to an external device.

5. The measuring device according to claim 4, wherein
the control unit is configured such that, even in a state in which the one identification code is written in only a single record and the other identification code is written in a plurality of records in common, the control unit instructs a communication I/F to transmit these records to the external device collectively.

6. The measuring device according to claim 4 or 5, wherein
the control unit is configured such that, when the communication I/F has transmitted all the records stored in the storage unit, the control unit erases all the records stored in the storage unit.

7. The measuring device according to any one of claims 1 to 6, further comprising a timer unit, wherein
a time data field capable of storing time data is further included in the record, and
the control unit is configured to write the time data that the timer unit outputs to the time data field in an associated manner with the measured data, together with writing of the measured data as a part of the record of the storage unit.

8. The measuring device according to any one of claims 1 to 7, wherein
the control unit includes an abnormal value monitoring unit,
an additional information field capable of storing additional information associated with the measured data is further included in the record, and
the abnormal value monitoring unit is configured to set an "abnormal value flag" in the additional information field of the record that corresponds to the measured data, when the measured data exceeds or falls below a predetermined reference value.

9. The measuring device according to any one of claims 1 to 8, wherein
the identification information is stored in an electronic tag that conforms to a near field communication (NFC) standard, and
the ID reading unit is configured to read the identification information from outside via near field communication that conforms to the NFC standard.

10. A measuring system comprising:
the measuring device according to any one of claims 1 to 9 that includes a communication I/F; and a terminal device connected to the measuring device through communication via the communication I/F, wherein
the measuring system is configured such that, in response to a predetermined command, the measuring device transmits a stored record, which is the record in which the measured data is written, to the terminal device, and the terminal device is configured to receive the stored record transmitted from the measuring device.

11. A method of controlling a measuring device for measuring an "amount to be measured" that is an amount indicating a state or a property of a measured object, wherein
the measuring device includes: a measuring unit configured to measure the amount to be measured;
an ID reading unit configured to read identification information from outside the measuring device, and configured to output the read identification information as an identification code;
a storage unit configured to store a plurality of records where a unit of a data structure, which includes at least: a measured data field capable of storing measured data obtained from a single measurement by the measurement unit; and an identification code field capable of storing the identification code associated with the measured data is defined as one "record"; and
a control unit configured to control an operation of the measuring device, wherein
the control unit includes:
a measured data accumulating step of executing, a set of a measuring process that instructs the measuring unit to measure the amount to be measured in response to an instruction of an operator, and, a measuring data writing process that writes the measured data obtained by the measurement as a part of the record in a predetermined field of the storage unit, one or more times;
an identification code assignment range selecting step of, when the ID acquisition unit newly reads the identification information and obtains the identification code, instructing the control unit to reference the storage unit and, when the storage unit is a state in which the identification code has not been written in an identification code field of the record that is one cycle prior to the record that corresponds to the most recent measurement cycle, prompting an operator to select either "(A) to assign an identification code to a single measured data" or "(B) to assign an identification code to a plurality of measured data"
an identification code individual assigning step of instructing the control unit, when the operator selects "(A) to assign an identification code to a single measured data", to write a newly obtained identification code only to the record that corresponds to the most recent measurement cycle and
an identification code bulk assigning step of instructing the control unit, when the operator selects "(B) to assign an identification code to a plurality of measured data", to collectively write the newly obtained same identification code to each consecutive record in which no identification code is written in the identification code field as viewed retroactively in order from the record of the most recent measurement cycle.
